# EUROPEAN PATENT SPECIFICATION

(11) **EP 0 571 390 B1**
(45) Date of publication and mention of the grant of the patent: **08.03.2000**
(21) Application number: 92900194.9
(22) Date of filing: 22.11.1991
(51) Int. Cl.: C12N 5/08, A61K 38/00, A61K 7/48

(54) **THE DELAY, PREVENTION AND/OR REVERSAL OF CELL SENESCENCE**
VERZÖGERUNG, VERHINDERUNG UND/ODER RÜCKGÄNGIGMACHEN VON ZELLALTERUNG
RETARDEMENT, PREVENTION ET/OU RENVERSEMENT DE LA SENESCENCE DE CELLULES

(30) Priority: 23.11.1990 AU 352490
(43) Date of publication of application: 01.12.1993
(73) Proprietor: Peptech Limited, Dee Why, NSW 2099 (AU); COMMONWEALTH SCIENTIFIC AND INDUSTRIAL RESEARCH ORGANISATION, Campbell, ACT 2612 (AU)
(72) Inventor: GRIGG, Geoffrey, Walter, Lane Cove, NSW 2066 (AU); HOLLIDAY, Robin, Hunters Hill, NSW 2110 (AU); McFARLAND, Gail, Annette, Westleigh, NSW 2120 (AU)
(74) Representative: Sheard, Andrew Gregory
(86) International application number: AU9100541
(87) International publication number: WO9209298

(56) References cited:
- EP-A- 0 060 565
- EP-A- 0 449 787
- WO-A-85/02342
- AU-A- 4 332 089
- US-A- 4 508 728
- DATABASE WPI Section Ch, Week 8725, Derwent Publications Ltd., London, GB; Class A96, AN 87-174442 'EXTERNALLY APPLICABLE COMPSNS. - CONTG. CLORPRENALINE HYDRO-CHLORIDE AND RELEASE-IMPROVER(S), USED IN TREATMENT OF BRONCHIAL ASTHMA,BRONCHITIS,ETC.' & JP-A-62 106 013 (KOWA KK) 16 May 1987
- PATENT ABSTRACTS OF JAPAN vol. 7, no. 205 (C-185)9 September 1983 & JP-A-58 103 317 (NITTO DENKI KOGYO KK) 20 June 1983
- PATENTS ABSTRACTS OF JAPAN, C201, page 88, JP,A,58 164 511 (KANESHIROU NAGAI) 29 September 1983 (29.09.83).
- PATENTS ABSTRACTS OF JAPAN, C201, page 90, JP,A,58 164 516 (KANESHIROU NAGAI) 29 September 1983 (29.09.83).
- PATENTS ABSTRACTS OF JAPAN, C780, page 38, JP,A,02 221 213 (KANESHIRO NAGAI) 4 September 1990 (04.09.90).
- PATENT ABSTRACTS OF JAPAN, C780, page 42, JP,A,02 221 230 (KANESHIRO NAGAI) 4 September 1990 (04.09.90).
- PATENT ABSTRACTS OF JAPAN, C711, page 44, JP,A,02 035 057 (KANESHIRO NAGAI) 5 February 1990 (05.02.90).
- CHEMICAL ABSTRACTS, Volume 104, No. 15, issued 1986, April 14 (Columbus, Ohio, USA), HAN, M.Y. et al., "The effect on L-carnosine of angiogenesis and the mode of action on cultured fibroblast", see page 463, column 1, Abstract No. 127222V, Han'guk Saenghwa Hakhoechi, 1985, 18(4), 423-8 (Korean).
- CHEMICAL ABSTRACTS, Volume 104, No. 15, issued 1986, April 14 (Columbus, Ohio, USA), HAN, M.Y. et al., "The effect of L-carnosine on proliferation and collagen synthesis of cultured fibroblast", see page 475, column 1, Abstract No. 127330d, Han'guk Saenghwa Hakhoechi, 1985, 18(4), 417-22 (Korean).

## Description

### Field of the Invention

The present invention relates to a method for the delay, prevention and/or reversal of cell senescence. In particular the invention has applicability to the delay, prevention and/or reversal of senescence in fibroblast cells. The present invention further relates to an improved cell culture medium.

### Background of the Invention

Carnosine is a dipeptide, β-alanyl-L-histidine, which is present in high amounts in tissues such as brain and muscle. Its function is unknown, but there are reports that it may act as an antioxidant and help protect cells from free radical damage. It has been shown that carnosine prevents collagen and protein crosslinking and this discovery was the basis for International Patent Application No WO90/06102 entitled "Compound and Method for the Retardation of Collagen Cross-Linking". Such cross-linking is well known to be associated with aging.

Human diploid fibroblasts constitute an important part of the skin. They secrete collagen, which is a structural component, and are active in wound healing. Small skin biopsies in normal culture medium yield primary cultures of dividing fibroblasts. These cells can be subcultured, or passaged, many times and they retain for a considerable period their characteristic morphology and diploid chromosome number.

The cells, however, eventually grow more slowly and an increasing proportion stop dividing. This is accompanied by significant morphological changes. Whereas young healthy fibroblasts are of uniform size and line up in parallel arrays to form characteristic "whorls" of cells in a confluent monolayer, cells which have divided many times in culture become more irregular in size, with many large cells, more granular in appearance, and they no longer form regular whorls. Such cells may grow slowly for several more divisions, but eventually cell division ceases and the population fails to form a confluent monolayer. At this time the cells are extremely heterogeneous in appearance, and many eventually detach from the substrate.

The limited life span of cultured human fibroblasts is sometimes referred to as the "Hayflick limit", after the discoverer of this phenomenon. Hayflick proposed that the cessation of growth was due to an intrinsic process which lead to terminal senescence, and that cultured human fibroblasts provide a suitable experimental model for the study of cellular ageing. His view has been confirmed by the finding that skin cells derived from biopsies from old individuals have a significantly shorter life span than those from young individuals. Also, in comparative studies with different mammalian species, it has been shown that the in vitro life span of their fibroblasts is directly related to the normal life span of each donor animal. Although innumerable investigations have documented in detail the limited in vitro life span of human fibroblasts and also other cell types, and many important experimental studies have been carried out, there is no agreement about the likely molecular basis of senescence and ageing in these cells.

It is known that carnosine aids growth and stimulates collagen synthesis in embryonic chick fibroblasts in culture (Han et al, 1985) and aids wound healing (Nagai et al, 1986). There are no prior claims however that carnosine affects ageing and senescence in tissue culture in the way described in this document. (references:- Han et al (1985) Korean Biochem. J. 18, 417-422; Nagai et al (1986) Surgery 100, 815-21).

The present inventors have discovered that the senescence of cells, particularly human fibroblast cells, may be delayed, prevented and/or reversed by maintaining the cells in the presence of an effective amount of a compound having chemical properties similar to carnosine. The chemical properties of carnosine include its ability to scavenge oxidative free radicals and its capacity to chelate certain cations including copper. Compounds having chemical properties similar to carnosine include carnosine, anserine, ophidine, homocarnosine, homoanserine, D-carnosine, carcinine, a compound of the formula R₁-L(or D)-His-R₂, cosmetically or pharmaceutically acceptable salts thereof and combinations thereof, in which R₁ = L-(or D)-lysine, or a homologue, optionally α-amino acylated with alkyl or aralkyl with 1 to 12 carbon atoms, preferably 2 to 6 carbon atoms, and R₂ = L-(or D)-lysine, or a homologue, optionally α-carboxyl esterified or amidated with alkyl or aralkyl with 1 to 12 carbon atoms, preferably 2 to 6 carbon atoms.

It has been well established by experiments performed by a number of independent groups that there is a close relationship between the passaging ability of human primary tissue culture cells and the tissue age of the donor. So, whilst a fibroblast culture established from tissues of a new born baby can be passaged through some 55 - 60 cell divisions before reaching its "Hayflick limit" and before dying, a similar culture established from a 60 year old can be passaged through only 10 or fewer cell divisions before dying. A culture established from a 30 year old has a "Hayflick limit" intermediate in size of between 10 and 60 cell divisions. The Hayflick limit of cells taken from a donor can be considered an index of the state of senescence of the tissues of the donor.

Ageing of human primary cell cultures are considered an accurate model of ageing of the whole human being.

Factors which affect ageing of cells in culture are believed of importance in ageing of tissues and the whole man. Generally, such studies have been used only to identity factors of which there are many which diminish survival expectations. Factors which prolong survival or which rejuvenate cells are exceptionally rare. The inventors know of only on other report where prolongation of human cell survival is well substantiated and that was for a non-peptide compound.

It will be obvious to those skilled in the biomedical science of ageing that a substance which extends the life of human primary fibroblasts in culture and which can rejuvenate senescent cultures which are close to their "Hayflick Limit" will be likely to have a similar effect in tissues in the whole human person - if delivered to that tissue at an appropriate rate and in an appropriate form.

Consequently, it is believed that a compound having chemical properties similar to carnosine would be useful in slowing down ageing in normal and diseased patients and rejuvenating such patients who already are senescent.

Moreover, they will have a beneficial effect on age-related diseases or conditions, such as Alzheimers Disease and other age-related neurodegenerative conditions. In addition such a compound should have application in cosmetics to rejuvenate skin and to assist the skin in its ability to renew itself and to slow down the aging process.

### Summary of the Invention

Accordingly, in a first aspect the present invention consists in a method of increasing the Hayflick limit of cells comprising applying to the cells an effective amount of a composition comprising carnosine, the active compound being present in the composition at a concentration of at least 10mM.

In a second aspect the present invention consists in a method of rejuvenating cells which have reached their Hayflick limit such that the cells will divide further, comprising applying to the cells an effective amount of a composition comprising carnosine as active compound, the active compound being present in the composition at a concentration of at least 10mM.

In a third aspect the present invention relates to the use of a composition comprising carnosine as active compound in the manufacture of an agent for increasing the Hayflick limit of cells, the active compound being present in the composition at a concentration of at least 10mM.

In a fourth aspect the present invention relates to the use of a composition comprising carnosine as active compound in the manufacture of an agent for rejuvenating cells which have reached their Hayflick limit such that the cells will divide further, the active compound being present in the composition at a concentration of at least 10mM.

The present invention does not cover matter excluded under Article 52(4) EPC.

It is preferred that the active compound is present in the composition in a concentration range of 10mM to 100mM, more preferably 10mM to 50mM and most preferably 15mM to 30mM.

In a further preferred embodiment of the present invention the cells are human fibroblast cells.

The composition may be administered to the cells in a culture medium in the case of cells being cultured in vitro. In the case of in vivo cells the composition may be applied in any suitable manner such as injection, infusion, ingestion, inhalation, iontophoresis, electroporation, or topical application.

It is presently preferred, however, that the composition is administered topically.

In a preferred embodiment the active compound(s) is mixed with or linked to another molecule(s), which molecule is such that the composition is improved in regard to skin penetration, skin application, tissue absorption/adsorption, skin sensitisation and/or skin irritation.

The molecule(s) is preferably selected from the group consisting of sodium lauryl sulphate, lauryl amonium oxide, ozone, decylmethyl sulphoxide, lauryl ethoxylate, octanol, dimethyl sulphoxide, propylene glycol, nitroglycerine, ethanol and combinations thereof.

The active compound may also be part of delivery system comprising fatty acids, lipids, liposomes, amino acids, peptides, polypeptides, proteins, carbohydrates, alcohols, polyalcohols, vitamins, anti-oxidants, preservatives, fragrances, organic or water based solvents and mixtures thereof.

In a further preferred embodiment of the present invention the composition includes a compound selected from the group consisting of bilirubin, carotenoids, mannitol, sorbitol, glutathione, selenium, uric acid, vitamin A, vitamin C, vitamin E and combinations thereof. It is also preferred that the pH of the composition is adjusted to optimise activity of the active compound and/or to improve tissue uptake.

In yet another preferred embodiment of the present invention the active compound is mixed with or linked to another molecule, which molecule is such that the composition is improved in regard to blood brain barrier penetration.

In a fifth aspect the present invention consists in an improved culture medium, the improvement comprising including in the cell culture medium an active compound having chemical properties similar to carnosine at a concentration greater than 10mM.

The invention will hereinafter be described with reference to carnosine for convenience, however, this is not to be taken as a limitation on the broad scope of the present invention.

### Detailed Description of the Invention

In order that the nature of the present invention may be more clearly understood, preferred forms thereof will now be described with reference to the following examples and figures in which:-
Figure 1 shows confluent young MRC-5 cells;
Figure 2 shows senescent MRC-5 cells;
Figure 3 shows RR cells grown continuously in 30mM carnosine;
Figure 4 shows RR cells growing continuously in 50mM carnosine;
Figure 5 shows growth of MRC-5 cells from early passage to senescence and no subsequent growth in normal medium (DMEM) and medium supplemented throughout with 20mM (0-0) and 30mM (x-x) carnosine. Control cells were also transferred to these concentrations of carnosine at the time indicated by the arrows (see Figure 8);
Figure 6 shows untreated senescent MRC-5 cells at PD level 61 which have reached the end of their lifespan;
Figure 7 shows cells growing continuously in 20mM carnosine at PD level 67;
Figure 8 shows the growth of MRC-5 cells after transfer from normal medium to medium containing 20mM (0-0) or 30mM (x-x) carnosine at PD level 55. The control cells (0-0) ceased growth at PD level 61 (dashed line);
Figure 9 shows MRC-5 cells grown in medium containing 30mM carnosine after transfer from normal medium at PD level 55. The ordinant indicates the yield of cells per flask at each sub-culture. The increase in cell yield corresponds to the increased growth rate shown in Figure 8;
Figure 10 shows control MRC-5 cells at 55 PDs beginning to show clear signs of senescence. These cells were transferred to 20mM and 30mM carnosine;
Figure 11 shows the control cells growing in 20mM carnosine 146 days after transfer at 64 PDs; and
Figure 12 shows control cells growing in 30mM carnosine 146 days after transfer at 63 PDs (photographed at lower magnification to show the whorls characteristic of young human fibroblasts).

### Effect of Carnosine on Human Fibroblast Growth and Lifespan RR Cells

Cells were grown in Dulbecco's modification of Eagles' Minimal Essential Medium, with 10% fetal calf serum. When cells became confluent and ceased division, they were harvested with trypsin - versene, and dispersed to form a suspension of single cells which was counted with a Coulter Counter. Depending on the cell yield the culture was split in a ratio of 1:2, 1:4, or 1:8. These split ratios correspond approximately to one, two or three cell population doublings when the new cultures become confluent. The exact number of population doublings is calculated from the cell yield at each sub-culture.

Initially, human foreskin fibroblasts were obtained from Dr Roger Reddel (Children's Medical Research Foundation) at passage 5, flasks were set up with differing concentrations of carnosine (10mM, 20mM, 30mM and 50mM) together with control cultures. Each of the cultures were passaged in the same medium.

These RR cells grew as rapidly as control cultures when 10mM and 20mM carnosine was added to standard medium. With 30mM there was some reduction in growth rate, and with 50mM a great reduction. After many population doublings, the cells grown in 30mM and 50mM retained their characteristic fibroblast morphology, that is, the cells are elongated, line up in parallel arrays to form characteristic whorls of growth, and exhibit contact inhibition of cell division.

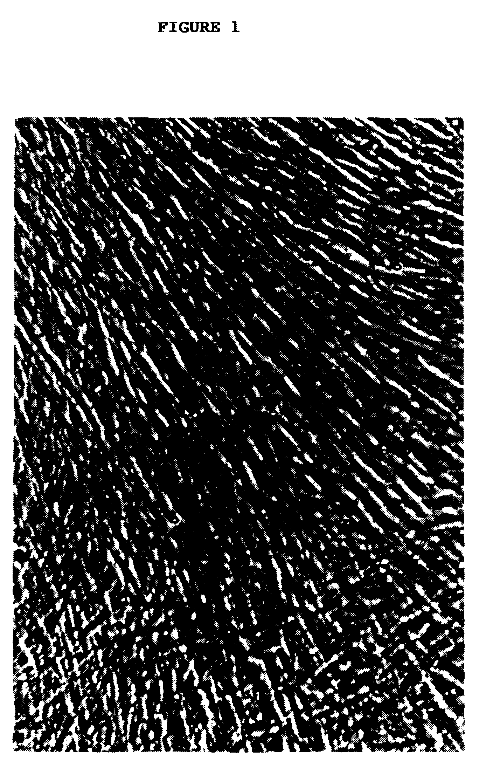

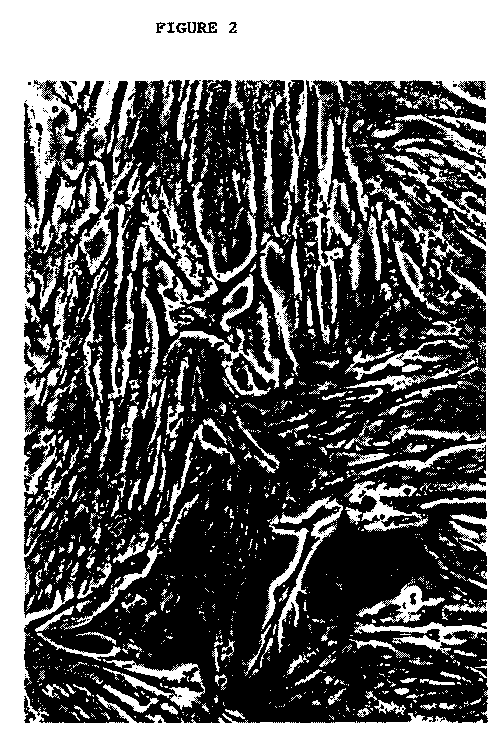

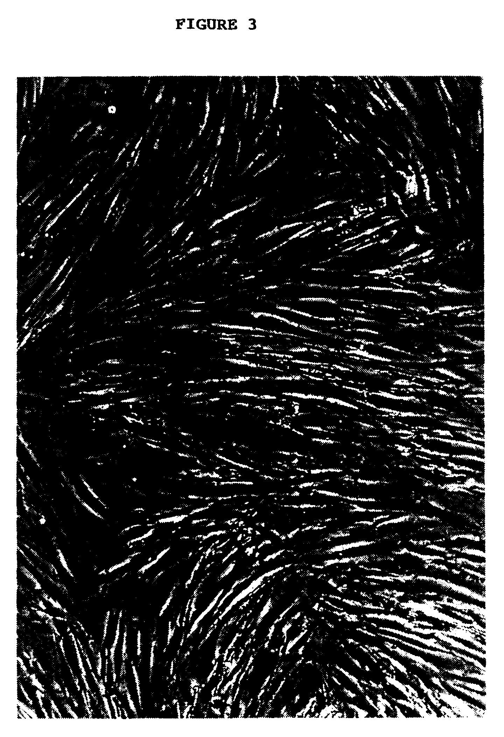

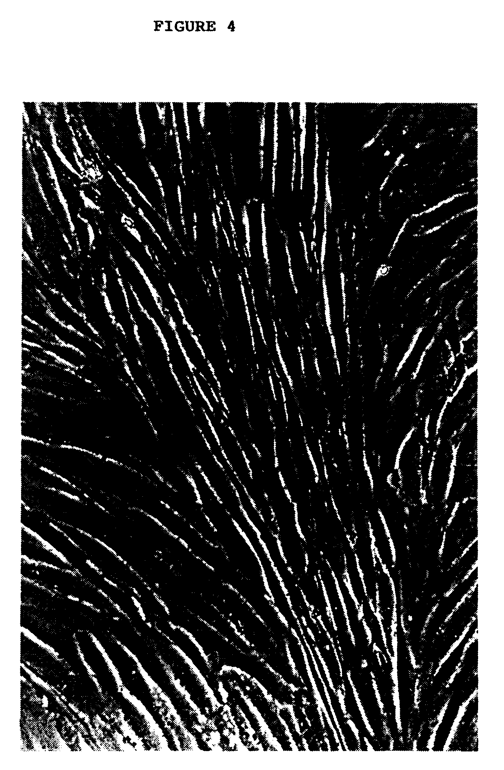

It is significant that the cultures in high levels of carnosine (30mM and 50mM) retained their fibroblast morphology, and are still growing after 500 days (Figures 3 and 4)(for comparison see Figures 1 and 2).

### MRC-5 cells

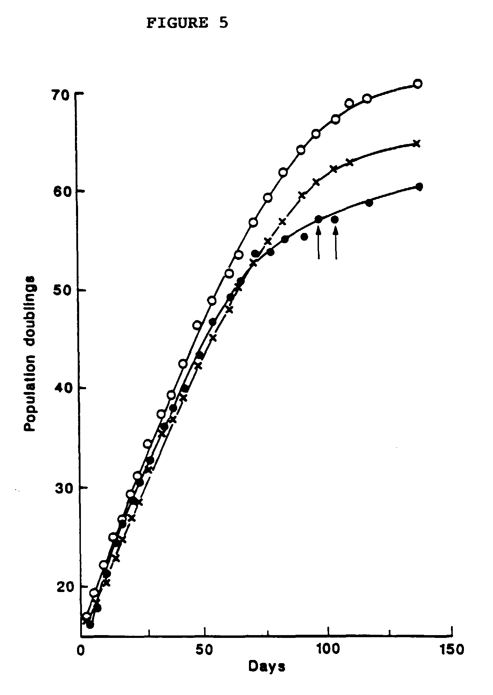

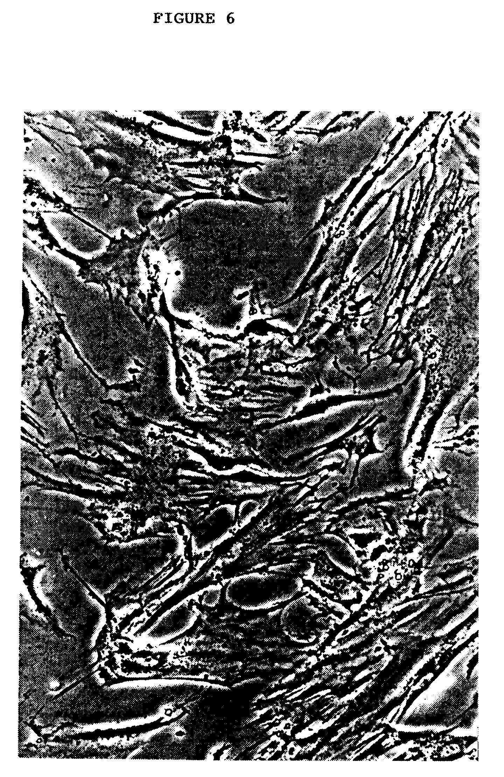

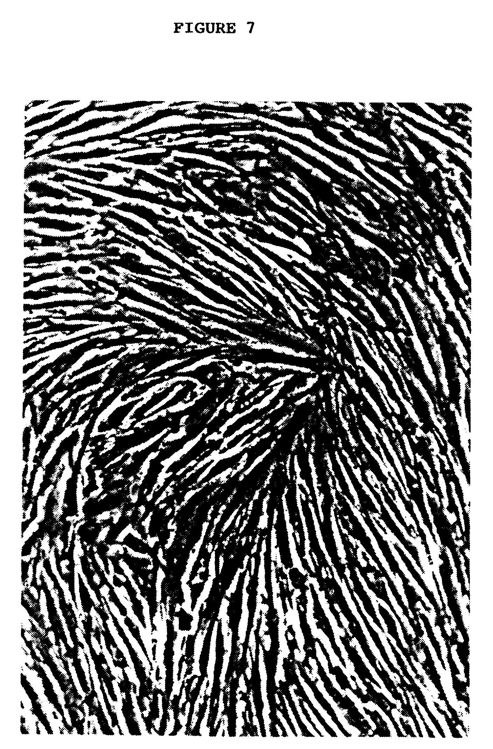

MRC-5 is a very well characterised fibroblast strain derived from foetal lung tissue. Early passage cultures were obtained from the National Institute for Biological Standards and Control, South Mimms, Herts, U.K. Longevity experiments were set up using normal medium and medium supplemented with 20 and 30mM carnosine (medium was the same as for RR cells above). There was little difference in growth rate in the initial stage. The control cells became senescent at the expected time, namely after about 50 - 55 PDs, and they ceased growth at 57 and 61 PDs. The cells in 20mM and 30mM carnosine had very significantly increased longevity (Table 1 and Figure 5). It should be noted that the PD level is a logarithmic measure of growth, so an increase of 10 PDs represents an increase in cell mass of 2¹⁰, or x 1,000. Carnosine also delayed the normal morphological features of senescence (Figure 7 in comparison to Figure 6). When control cells become senescent, the yield of cells per flask declines, but carnosine significantly increases the number of cells per flask as the end of the culture lifespan approaches.

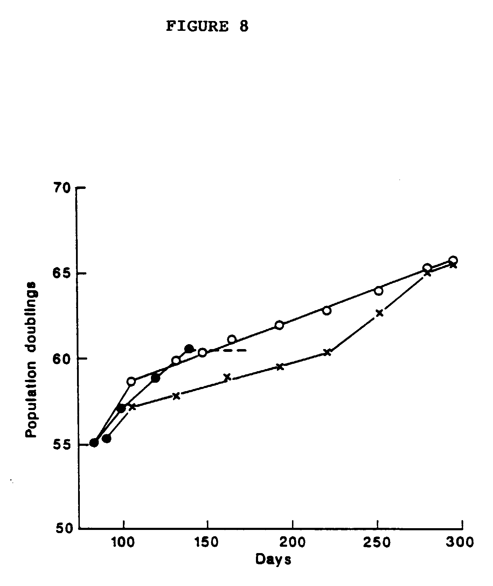

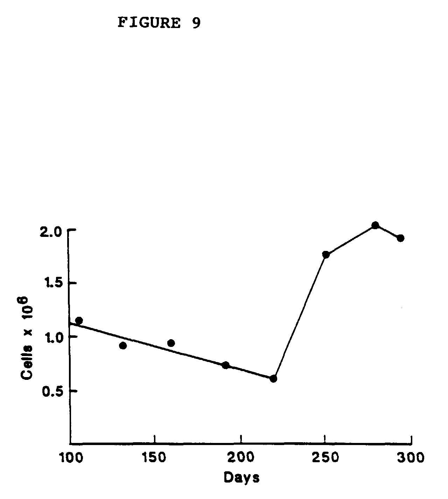

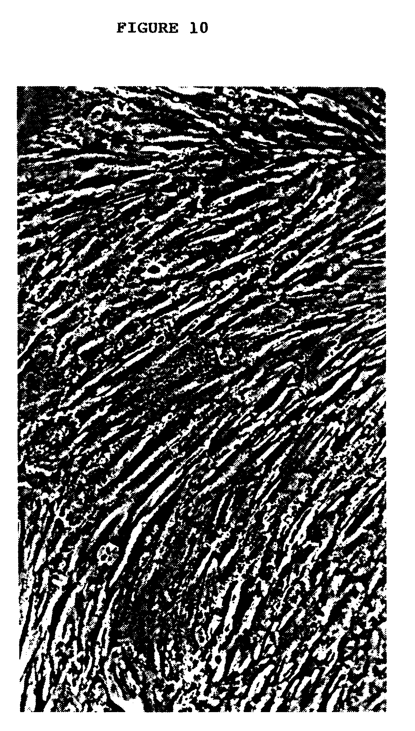

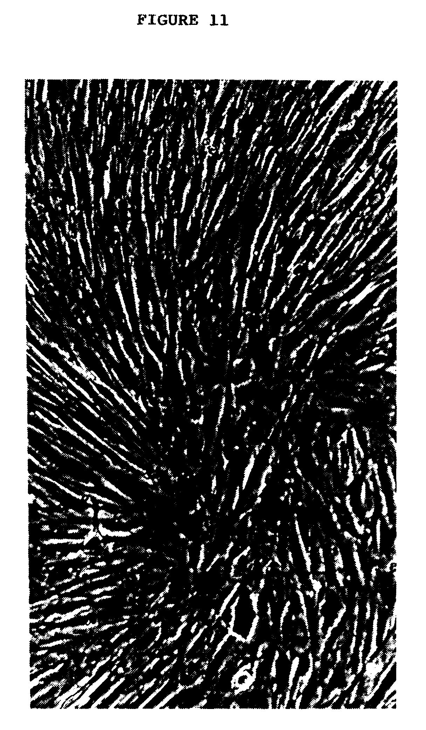

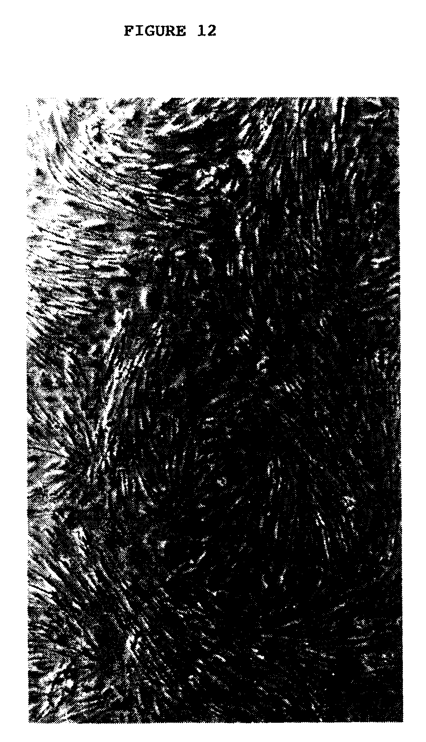

Untreated cells which had slowed down and exhibited a senescent phenotype were transferred at PD level 55 to 20mM and 30mM carnosine. These cultures continued to grow slowly, but with a rejuvenated phenotype. Remarkably, these cells are continuing to grow after 400 days (Figure 8), whereas cells grown continuously in carnosine died out after 139 days. Thus it appears that transfer of senescent cells to carnosine can reverse the senescent phenotype and allow extended growth of cells with a near normal morphology (Figures 10, 11 and 12). The effect of carnosine in increasing cell yield was particularly striking in cells transferred from normal medium to 30mM carnosine medium (Figure 9).

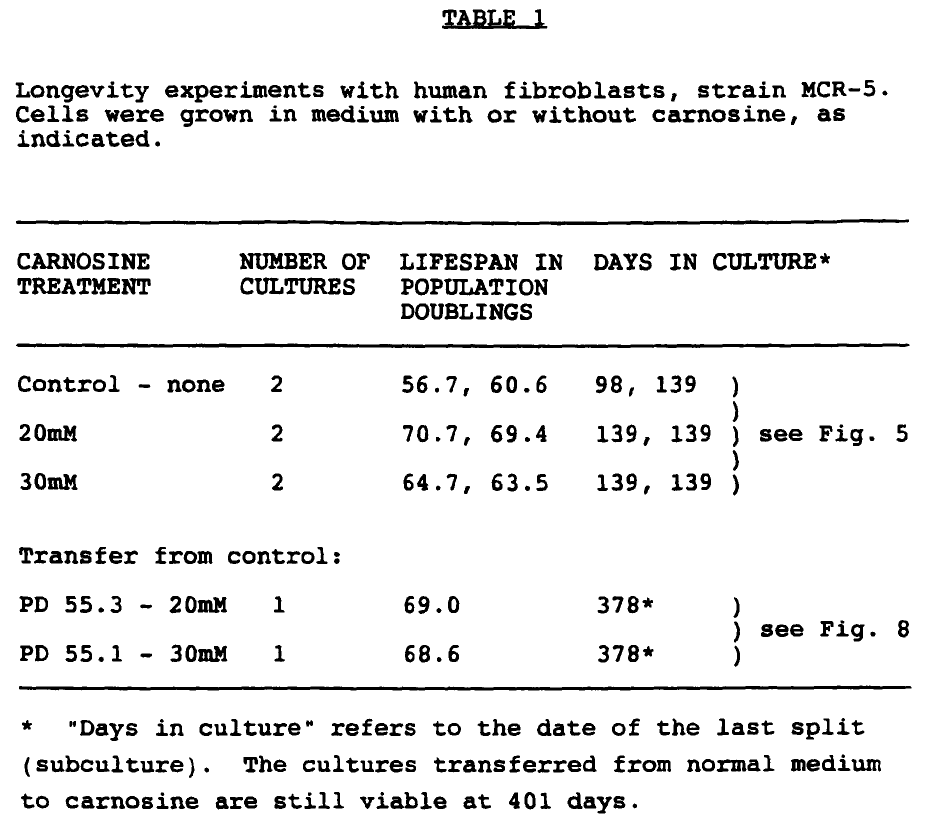

As will be clear from the above results the present inventors have shown that senescence of cells may be delayed, prevented and/or reversed by the administration of carnosine.

In addition an improved cell culture medium is provided which includes carnosine at a concentration of greater than 10mM. This culture medium can be of any of the large range of culture media known to persons skilled in this field to which carnosine has been added.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

## Claims

1. A method of increasing the Hayflick limit of cells in culture comprising applying to the cells an effective amount of a composition comprising carnosine as active compound, the active compound being present in the composition at a concentration of at least 10 mM.

2. A method of rejuvenating cells in culture which have reached their Hayflick limit such that the cells will divide further, comprising applying to the cells an effective amount of a composition comprising carnosine as active compound, the active compound being present in the composition at a concentration of at least 10 mM.

3. A cosmetic method of increasing the Hayflick limit of cells, or rejuvenating cells which have reached their Hayflick limit such that the cells will divide further, comprising applying to the cells an effective amount of a composition comprising carnosine as the active compound, the active compound being present in the composition at a concentration of at least 10mM.

4. A method as claimed in claim 1, 2 or 3, in which the active compound is present in a concentration of between 10 mM and 100 mM.

5. A method as claimed in claim 1, 2 or 3, in which the active compound is present in a concentration of between 10 mM and 50 mM.

6. A method as claimed in claim 5 in which the active compound is present at a concentration of between 15 mM and 30 mM.

7. A method as claimed in any one of claims 1 to 6 in which the cells are human fibroblast cells.

8. Use of a composition comprising carnosine as active compound in the manufacture of an agent for increasing the Hayflick limit of cells, the active compound being present in the composition at a concentration of at least 10 mM.

9. The use of a composition comprising carnosine as active compound in the manufacture of an agent for rejuvenating cells which have reached their Hayflick limit such that the cells will divide further, the active compound being present in the composition at a concentration of at least 10 mM.

10. The use according to claims 8 or 9 wherein the active compound is present at a concentration of between 10 mM and 100 mM.

11. The use according to claims 8 or 9 wherein the active compound is present at a concentration of between 10 mM and 50 mM.

12. The use according to claims 8 or 9 wherein the active compound is present at a concentration of between 15 mM and 30 mM.

13. The use according to any one of claims 8 to 12 wherein the cells are human fibroblast cells.

## Patentansprüche

1. Verfahren zum Erhöhen des Hayflick-Limits von Zellen in Kultur, umfassend Behandeln der Zellen mit einer wirksamen Menge einer Zusammensetzung, umfassend Carnosin als Wirkstoff, wobei der Wirkstoff in der Zusammensetzung in einer Konzentration von mindestens 10 mM vorhanden ist.

2. Verfahren zur Verjüngung von Zellen in Kultur, welche ihr Hayflick-Limit erreicht haben, dergestalt daß die Zellen sich weiter teilen, umfassend Behandeln der Zellen mit einer wirksamen Menge einer Zusammensetzung, umfassend Carnosin als Wirkstoff, wobei der Wirkstoff in der Zusammensetzung in einer Konzentration von mindestens 10 mM vorhanden ist

3. Kosmetisches Verfahren zur Erhöhung des Hayflick-Limits von Zellen, oder zur Verjüngung von Zellen, welche ihr Hayflick-Limit erreicht haben, dergestalt daß die Zellen sich weiter teilen, umfassend Behandeln der Zellen mit einer wirksamen Menge einer Zusammensetzung, umfassend Carnosin als Wirkstoff, wobei der Wirkstoff in der Zusammensetzung in einer Konzentration von mindestens 10 mM vorhanden ist.

4. Verfahren nach einem der Ansprüche 1, 2 oder 3, worin der Wirkstoff in einer Konzentration von zwischen 10 mM und 100 mM vorhanden ist.

5. Verfahren nach einem der Ansprüche 1, 2 oder 3, worin der Wirkstoff in einer Konzentration von zwischen 10 mM und 50 mM vorhanden ist.

6. Verfahren nach Anspruch 5, worin der Wirkstoff in einer Konzentration von zwischen 15 mM und 30 mM vorhanden ist.

7. Verfahren nach einem der Ansprüche 1 bis 6, worin die Zellen humane Fibroblastenzellen sind.

8. Verwendung einer Zusammensetzung, umfassend Carnosin als Wirkstoff, zur Herstellung eines Mittels zur Erhöhung des Hayflick-Limits von Zellen, wobei der Wirkstoff in der Zusammensetzung in einer Konzentration von mindestens 10 mM vorhanden ist.

9. Verwendung einer Zusammensetzung, umfassend Carnosin als Wirkstoff, zur Herstellung eines Mittels zur Verjüngung von Zellen, welche ihr Hayflick-Limit erreicht haben, dergestalt daß die Zellen sich weiter teilen, wobei der Wirkstoff in der Zusammensetzung in einer Konzentration von mindestens 10 mM vorhanden ist.

10. Verwendung nach Anspruch 8 oder 9, worin der Wirkstoff in einer Konzentration von zwischen 10 mM und 100 mM vorhanden ist

11. Verwendung nach Anspruch 8 oder 9, worin der Wirkstoff in einer Konzentration von zwischen 10 mM und 50 mM vorhanden ist.

12. Verwendung nach Anspruch 8 oder 9, worin der Wirkstoff in einer Konzentration von zwischen 15 mM und 30 mM vorhanden ist.

13. Verwendung nach einem der Ansprüche 8 bis 12, worin die Zellen humane Fibroblastenzellen sind.

## Revendications

1. Procédé d'augmentation de la limite de Hayflick de cellules en culture, comprenant l'application aux cellules d'une quantité efficace d'une composition comprenant de la carnosine à titre de composé actif, le composé actif étant présent dans la composition à une concentration d'au moins 10 mM.

2. Procédé de rajeunissement de cellules en culture qui ont atteint leur limite de Hayflick de façon que les cellules se divisent encore, comprenant l'application aux cellules d'une quantité efficace d'une composition comprenant de la carnosine à titre de composé actif, le composé actif étant présent dans la composition à une concentration d'au moins 10 mM.

3. Procédé cosmétique d'augmentation de la limite de Hayflick de cellules, ou de rajeunissement de cellules qui ont atteint leur limite de Hayflick de façon que les cellules se divisent encore, comprenant l'application aux cellules d'une quantité efficace d'une composition comprenant de la carnosine à titre de composé actif, le composé actif étant présent dans la composition à une concentration d'au moins 10 mM.

4. Procédé selon la revendication 1, 2 ou 3, dans lequel le composé actif est présent à une concentration comprise entre 10 mM et 100 mM.

5. Procédé selon la revendication 1, 2 ou 3, dans lequel le composé actif est présent à une concentration comprise entre 10 mM et 50 mM.

6. Procédé selon la revendication 5, dans lequel le composé actif est présent à une concentration comprise entre 15 mM et 30 mM.

7. Procédé selon l'une quelconque des revendications 1 à 6, dans lequel les cellules sont des cellules fibroblastes humaines.

8. Utilisation d'une composition comprenant de la carnosine à titre de composé actif dans la fabrication d'un agent qui éleve la limite de Hayflick de cellules, le composé actif étant présent dans la composition à une concentration d'au moins 10 mM.

9. Utilisation d'une composition comprenant de la carnosine à titre de composé actif dans la fabrication d'un agent pour rajeunir des cellules qui ont atteint leur limite de Hayflick de façon que les cellules se divisent encore, le composé actif étant présent dans la composition à une concentration d'au moins 10 mM.

10. Utilisation selon la revendication 8 ou 9, dans laquelle le composé actif est présent à une concentration entre 10 mM et 100 mM.

11. Utilisation selon la revendication 8 ou 9, dans laquelle le composé actif est présent à une concentration entre 10 mM et 50 mM.

12. Utilisation selon la revendication 8 ou 9, dans laquelle le composé actif est présent à une concentration entre 15 mM et 30 mM.

13. Utilisation selon l'une quelconque des revendications 8 à 12, dans laquelle les cellules sont des cellules fibroblastes humaines.
